# EUROPEAN PATENT APPLICATION

(11) **EP 4 767 928 A1**
(43) Date of publication of application: **01.07.2026**
(21) Application number: 24915572.2
(22) Date of filing: 19.12.2024
(51) Int. Cl.: A61B 5/024, A61B 5/00, A61B 5/11

(54) **ELECTRONIC DEVICE FOR DETERMINING SLEEPING HEART RATE AND/OR SLEEPING HEART RATE VARIABILITY, AND METHOD FOR OPERATING ELECTRONIC DEVICE**

(30) Priority: 05.01.2024 KR 20240002418; 17.01.2024 KR 20240007434
(71) Applicant: Samsung Electronics Co., Ltd., Suwon-si, Gyeonggi-do 16677 (KR)
(72) Inventor: CHO, Sunghwan, Suwon-si, Gyeonggi-do 16677 (KR); LEE, Dasom, Suwon-si, Gyeonggi-do 16677 (KR); LEE, Jaehun, Suwon-si, Gyeonggi-do 16677 (KR); JANG, Hojin, Suwon-si, Gyeonggi-do 16677 (KR)
(74) Representative: Gulde & Partner
(86) International application number: PCT/KR2024/020742
(87) International publication number: WO 2025/146990

(57) **Abstract**

An electronic device is provided and is communicative with an external electronic device. The electronic device includes a processor and a memory storing instructions. When the processor executes the instructions, the electronic device receives a user's heart rate (HR) and/or heart rate variability (HRV) from the external electronic device, maps the HR and/or the HRV to the user's sleep state, selects one of the HR measured in a first state among the sleep states and the HR measured between a designated time from the user's wake up time and the wake up time as a sleep HR, selects one of the HRV measured in the first state among the sleep states and the HRV measured between the designated time from the user's wake up time and the wake up time as sleep HRV and performs at least one operation based on the sleep HR and/or the sleep HRV.

## Description

### [Technical Field]

The disclosure relates to an electronic device and a method of operating the electronic device, and relates to technology for determining a sleep heart rate and/or sleep heart rate variability.

### [Background Art]

Various electronic devices such as smart phones, tablet personal computers (PCs), portable multimedia players (PMPs), personal digital assistants (PDAs), laptop personal computers (laptop PCs), and/or wearable devices are becoming widespread.

Electronic devices implemented in the form of wearable devices may be implemented or configured to collect various biometric information of users. A user's biometric information may include at least one of the user's heart rate (HR), heart rate variability, electrocardiogram (ECG), blood pressure, or oxygen saturation.

An electronic device implemented in the form of a wearable device for collecting biometric information of a user may acquire the user's biometric information using various types of sensors (e.g., biometric optical sensor or electrocardiogram sensor) included therein.

### [Disclosure of Invention]

### [Technical Problem]

A user's biometric information collected by an electronic device may include a heart rate and/or heart rate variability. The heart rate may refer to the number of heart beats in a minute. The heart rate variability may be an indicator of changes in the time interval between heart beats.

Especially, a heart rate and/or heart rate variability measured while the user is sleeping may be used as indicators of a sleep quality and the user's recovery from fatigue through sleep.

A heart rate and/or heart rate variability measured during sleep may vary significantly, and some heart rates and/or heart rate variability may not be representative of the user's heart rate and/or heart rate variability measured during sleep.

Information related to the user's body created (or provided) using a heart rate and/or heart rate variability that cannot represent the user's physical condition may have low accuracy.

Technical problems to be achieved in this document are not limited to the above-described technical problems, and other technical problems not described will be clearly understood by those skilled in the art from the description below.

### [Solution to Problem]

According to an embodiment, an electronic device may include a communication circuit configured to communicate with an external electronic device configured to measure movements and/or bio-signals of a user thereof. The electronic device may include a processor. The electronic device may include memory. When executed by the processor, the memory may store an instruction for causing the electronic device to receive the user's heart rate (HR) and/or heart rate variability (HRV) measured by the external electronic device. The memory may store an instruction for causing the electronic device to map and store the heart rate and/or the heart rate variability to the user's sleep state determined based on the user's movement measured by the external electronic device. The memory may store an instruction for causing the electronic device to select one of the heart rate measured in a first state among the sleep states and a heart rate measured between a time point before a designated time from the user's wake up time and the wake up time as a sleep heart rate. The memory may store an instruction for causing the electronic device to select one of the heart rate variability measured in a first state among the sleep states and heart rate variability measured between a time point before a designated time from the user's wake up time and the wake up time as sleep heart rate variability. The memory may store an instruction for causing the electronic device to perform at least one operation based on the sleep heart rate and/or the sleep heart rate variability.

According to an embodiment, a method of operating an electronic device may include receiving the user's heart rate (HR) and/or heart rate variability measured by an external electronic device configured to measure the user's movements and/or bio-signals of the electronic device. The method of operating an electronic device may include mapping the heart rate and/or the heart rate variability to the user's sleep state determined based on the user's movement measured by the external electronic device and storing the heart rate and/or the heart rate variability in a memory. The method of operating an electronic device may include selecting one of the heart rate measured in a first state among the sleep states and a heart rate measured between a time point before a time designated from the user's wake up time and the wake up time as a sleep heart rate. The method of operating an electronic device may include selecting one of the heart rate variability measured in a first state among the sleep states and heart rate variability measured between a time point before a designated time from the user's wake up time and the wake up time as sleep heart rate variability. The method of operating an electronic device may include performing at least one operation based on the sleep heart rate and/or the sleep heart rate variability.

According to an embodiment, when executed by a processor of an electronic device, in a computer readable recording medium for storing instructions for causing the electronic device to perform an electronic device, the instructions may include an instruction for causing the electronic device to receive a user's heart rate (HR) and/or heart rate variability measured by an external electronic device. The instructions may include an instruction for causing the electronic device to map and store the heart rate and/or heart rate variability to the user's sleep state determined based on the user's movement measured by the external electronic device. The instructions may include an instruction for causing the electronic device to select one of the heart rate measured in a first state among the sleep states and a heart rate measured between a time point before a designated time from the user's wake up time and the wake up time as a sleep heart rate. The instructions may include an instruction for causing the electronic device to select one of the heart rate variability measured in a first state among the sleep states and heart rate variability measured between a time point before a designated time from the user's wake up time and the wake up time as sleep heart rate variability. The instructions may include an instruction for causing the electronic device to perform at least one operation based on the sleep heart rate and/or the sleep heart rate variability.

According to an embodiment, an electronic device includes a communication circuit configured to communicate with an external electronic device, a processor and a memory having executable instructions stored thereon. When the executable instructions are executed by the processor, the electronic device is caused to execute a method. The method includes receiving a heart rate (HR) and heart rate variability (HRV) of a user measured by the external electronic device, determining sleep states of the user based on user movement measured by the external electronic device, mapping the HR and the HRV to the sleep states, selecting, as a sleep HR, one of the HR measured in a first state among the sleep states and the HR measured between a designated time and a wake-up time of the user, selecting, as a sleep HRV, one of the HRV measured in the first state among the sleep states and the HRV measured between the designated time and the wake-up time and performing an operation based on the sleep HR and the sleep HRV.

### [Advantageous Effects of Invention]

In an electronic device and a method of operating the electronic device according to an embodiment, the electronic device may determine any one of a heart rate measured in a first state (e.g., during deep sleep) and a heart rate (or average of heart rates) measured from a time point before a designated time (e.g., 5 to 30 minutes) from a user's wake up time to the wake up time as a sleep heart rate. The electronic device may determine any one of heart rate variability measured in a first state (e.g., deep sleep state) and heart rate variability (or average of heart rate variability) measured from a time point before a designated time (e.g., 5 to 30 minutes) from the user's wake up time to the wake up time as sleep heart rate variability. The electronic device may determine an accurate heart rate and/or heart rate variability of a user (e.g., a user who has a lot of activity amount just before sleep) with a specific behavioral pattern, and provide information related to the user's body generated (or provided) using a sleep heart rate and/or sleep heart rate variability that may represent the user's physical condition, thereby helping improve the user's physical abilities.

Effects that can be obtained from the disclosure are not limited to the above-described effects, and other effects not described will be clearly understood by those of ordinary skill in the art to which the disclosure belongs from the description below.

### [Brief Description of Drawings]

FIG. 1 is a block diagram illustrating an electronic device according to various embodiments of the disclosure.
FIG. 2 is a block diagram illustrating a program according to an embodiment.
FIG. 3 is a block diagram illustrating an electronic device and an external electronic device according to an embodiment.
FIG. 4 is a block diagram illustrating an electronic device according to an embodiment.
FIG. 5A is a graph illustrating heart rate variability according to sleep stages in an electronic device according to an embodiment.
FIG. 5B is a graph illustrating sequential heart rate variability in an electronic device according to an embodiment.
FIG. 6 is a block diagram illustrating an external electronic device according to an embodiment.
FIG. 7 is a block diagram illustrating an electronic device according to an embodiment.
FIG. 8A, 8B, and/or 8C are diagrams illustrating an electronic device performing at least one operation based on a sleep heart rate and/or sleep heart rate variability according to an embodiment.
FIG. 9 is a flowchart illustrating a method of operation an electronic device according to an embodiment.

### [Mode for the Invention]

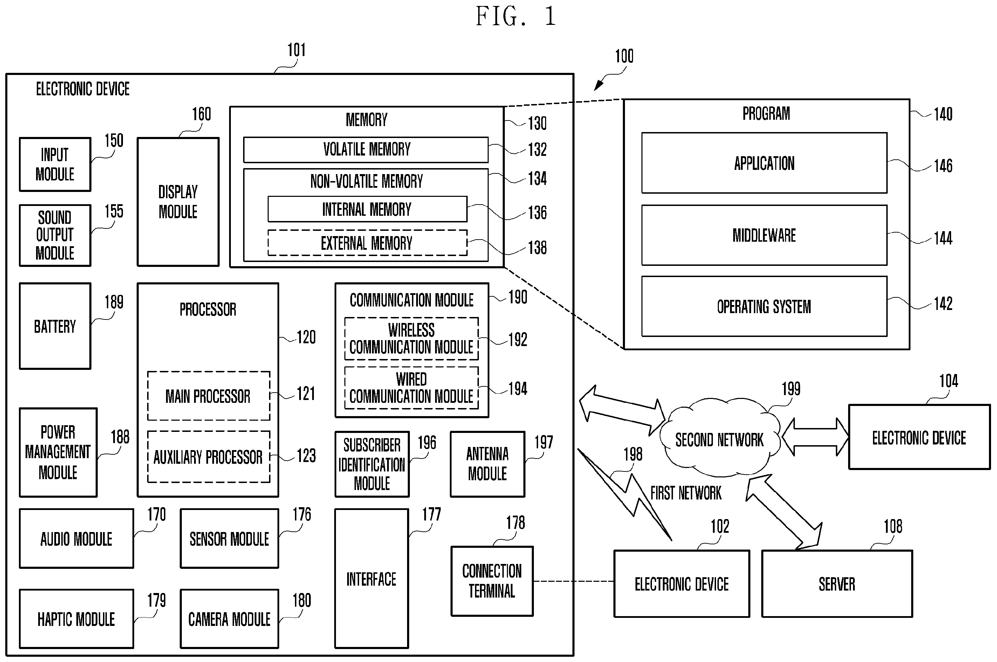

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output device 155, a display module 160, an audio module 170, a sensor module 176, an interface 177, a connection terminal 178, a haptic module 179, a camera module 180, a power management module 188, a battery 189, a communication module 190, a subscriber identification module (SIM) 196, or an antenna module 197. In some embodiments, at least one of the components (e.g., the connection terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output device 155 may output sound signals to the outside of the electronic device 101. The sound output device 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output device 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connection terminal 178 may include a connector via which the electronic device 101 may be physically connected with the external electronic device (e.g., the electronic device 102). According to an embodiment, the connection terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

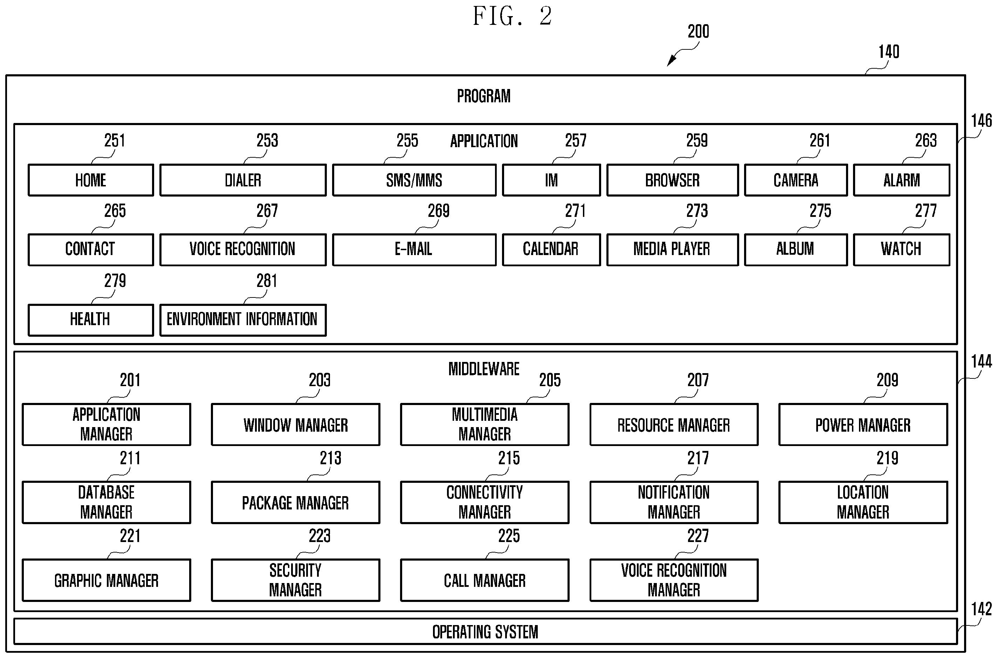

FIG. 2 is a block diagram 200 illustrating the program 140 according to various embodiments. According to an embodiment, the program 140 may include an operating system (OS) 142 to control one or more resources of the electronic device 101, middleware 144, or an application 146 executable in the OS 142. The OS 142 may include, for example, AndroidTM, iOSTM, WindowsTM, SymbianTM, TizenTM, or BadaTM. At least part of the program 140, for example, may be pre-loaded on the electronic device 101 during manufacture, or may be downloaded from or updated by an external electronic device (e.g., the electronic device 102 or 104, or the server 108) during use by a user.

The OS 142 may control management (e.g., allocating or deallocation) of one or more system resources (e.g., process, memory, or power source) of the electronic device 101. The OS 142, additionally or alternatively, may include one or more driver programs to drive other hardware devices of the electronic device 101, for example, the input module 150, the sound output device 155, the display module 160, the audio module 170, the sensor module 176, the interface 177, the haptic module 179, the camera module 180, the power management module 188, the battery 189, the communication module 190, the subscriber identification module 196, or the antenna module 197.

The middleware 144 may provide various functions to the application 146 such that a function or information provided from one or more resources of the electronic device 101 may be used by the application 146. The middleware 144 may include, for example, an application manager 201, a window manager 203, a multimedia manager 205, a resource manager 207, a power manager 209, a database manager 211, a package manager 213, a connectivity manager 215, a notification manager 217, a location manager 219, a graphic manager 221, a security manager 223, a telephony manager 225, or a voice recognition manager 227.

The application manager 201, for example, may manage the life cycle of the application 146. The window manager 203, for example, may manage one or more graphical user interface (GUI) resources that are used on a screen. The multimedia manager 205, for example, may identify one or more formats to be used to play media files, and may encode or decode a corresponding one of the media files using a codec appropriate for a corresponding format selected from the one or more formats. The resource manager 207, for example, may manage the source code of the application 146 or a memory space of the memory 130.The power manager 209, for example, may manage the capacity, temperature, or power of the battery 189, and determine or provide related information to be used for the operation of the electronic device 101 based at least in part on corresponding information of the capacity, temperature, or power of the battery 189. According to an embodiment, the power manager 209 may interwork with a basic input/output system (BIOS) (not shown) of the electronic device 101.

The database manager 211, for example, may generate, search, or change a database to be used by the application 146. The package manager 213, for example, may manage installation or update of an application that is distributed in the form of a package file. The connectivity manager 215, for example, may manage a wireless connection or a direct connection between the electronic device 101 and the external electronic device. The notification manager 217, for example, may provide a function to notify a user of an occurrence of a specified event (e.g., an incoming call, message, or alert). The location manager 219, for example, may manage locational information on the electronic device 101. The graphic manager 221, for example, may manage one or more graphic effects to be offered to a user or a user interface related to the one or more graphic effects.

The security manager 223, for example, may provide system security or user authentication. The telephony manager 225, for example, may manage a voice call function or a video call function provided by the electronic device 101. The voice recognition manager 227, for example, may transmit a user's voice data to the server 108, and receive, from the server 108, a command corresponding to a function to be executed on the electronic device 101 based at least in part on the voice data, or text data converted based at least in part on the voice data. According to an embodiment, the middleware 244 may dynamically delete some existing components or add new components. According to an embodiment, at least part of the middleware 144 may be included as part of the OS 142 or may be implemented as another software separate from the OS 142.

The application 146 may include, for example, a home 251, dialer 253, short message service (SMS)/multimedia messaging service (MMS) 255, instant message (IM) 257, browser 259, camera 261, alarm 263, contact 265, voice recognition 267, email 269, calendar 271, media player 273, album 275, watch 277, health 279 (e.g., for measuring the degree of workout or biometric information, such as blood sugar), or environmental information 281 (e.g., for measuring air pressure, humidity, or temperature information) application. According to an embodiment, the application 146 may further include an information exchanging application (not shown) that is capable of supporting information exchange between the electronic device 101 and the external electronic device. The information exchange application, for example, may include a notification relay application adapted to transfer designated information (e.g., a call, message, or alert) to the external electronic device or a device management application adapted to manage the external electronic device. The notification relay application may transfer notification information corresponding to an occurrence of a specified event (e.g., receipt of an email) at another application (e.g., the email application 269) of the electronic device 101 to the external electronic device. Additionally or alternatively, the notification relay application may receive notification information from the external electronic device and provide the notification information to a user of the electronic device 101.

The device management application may control the power (e.g., turn-on or turn-off) or the function (e.g., adjustment of brightness, resolution, or focus) of the external electronic device or some component thereof (e.g., a display module or a camera module of the external electronic device). The device management application, additionally or alternatively, may support installation, delete, or update of an application running on the external electronic device.

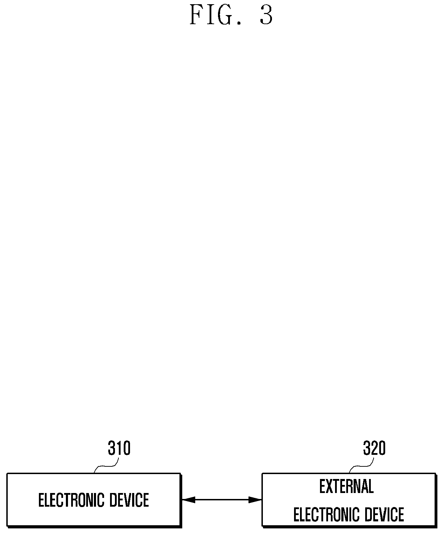

FIG. 3 is a block diagram illustrating an electronic device and an external electronic device according to an embodiment.

With reference to FIG. 3, the electronic device 310 (e.g., the electronic device 101 of FIG. 1) may be an electronic device that acquires a user's biometric information and that performs various operations based on the biometric information.

According to an embodiment, the user's biometric information may include information related to the user's health. For example, the user's biometric information may include at least one of the user's heart rate (HR), heart rate variability, electrocardiogram (ECG), blood pressure, or oxygen saturation. The user's biometric information may include various types of information in addition to the examples described above.

The heart rate may refer to the number of heart beats in a minute. The heart rate variability may be an indicator of changes in the time interval between heart beats. The electrocardiogram may refer to an electrical signal of the heart according to the contraction and/or relaxation of the heart. The blood pressure may refer to a pressure applied to blood vessels due to the movement of blood. The oxygen saturation may refer to the extent to which hemoglobin in the blood includes oxygen.

The electronic device 310 may acquire the user's biometric information using various sensors (e.g., biometric optical sensor, electrocardiogram sensor) included therein.

According to an embodiment, the electronic device 310 may acquire the user's biometric information using an external electronic device 320 wirelessly connected thereto. The external electronic device 320 may be a wearable electronic device that may be worn by a user of the electronic device 310 or the external electronic device 320. For example, the external electronic device 320 may be an electronic device (e.g., smart watch or smart ring) worn on the user's body (e.g., user's wrist, user's finger).

The external electronic device 320 may acquire the user's biometric information using various sensors (e.g., biometric optical sensor, electrocardiogram sensor) included therein and transmit the user's acquired biometric information to the electronic device 310 through short-range wireless communication (e.g., BLE, Wi-Fi, Zigbee).

The electronic device 310 may perform at least one operation based on the user's biometric information acquired thereby and/or the user's biometric information acquired by the external electronic device 320.

According to an embodiment, the electronic device 310 may generate guide information that may assist the user's physical abilities based on the user's biometric information acquired thereby and/or the user's biometric information acquired by the external electronic device 320 and/or provide the guide information to the user in various ways (e.g., display on the display, output through the speaker).

According to an embodiment, the electronic device 310 may generate information related to the user's vitality based on the user's biometric information acquired thereby and/or the user's biometric information acquired by the external electronic device 320. Information related to the user's vitality may be implemented in the form of a number or a text. For example, information related to the user's vitality may include a number (or score) indicating the user's level of vitality.

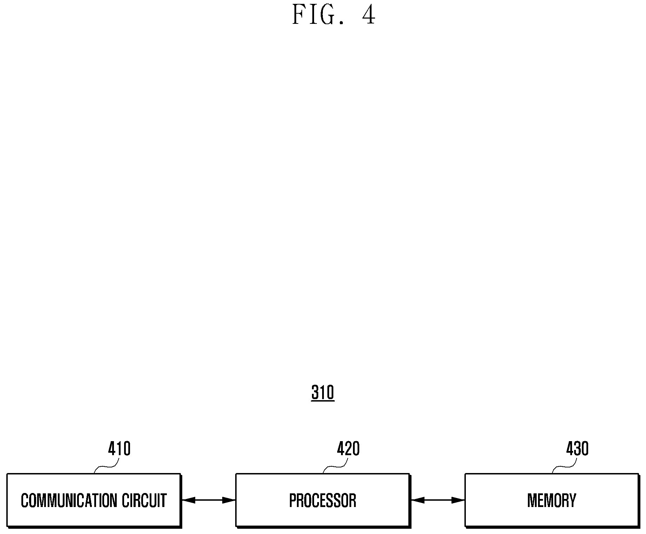

FIG. 4 is a block diagram illustrating an electronic device according to an embodiment.

According to an embodiment, the electronic device (e.g., the electronic device 310 of FIG. 3) may include a communication circuit 410 (e.g., the wireless communication module 192 of FIG. 1), a processor 420 (e.g., the processor 120 of FIG. 1), and/or a memory 430 (e.g., the memory 130 of FIG. 1).

The communication circuit 410 may include various circuit structures used for modulating and/or demodulating signals within the electronic device 310. For example, the communication circuit 410 may modulate a baseband signal into a radio frequency (RF) band signal so as to output through an antenna (not illustrated) or may demodulate an RF band signal received through an antenna into a baseband signal and transmit the baseband signal to the processor 420. The communication circuit 410 may support short-range wireless communication (e.g., Bluetooth, low-power Bluetooth, Wi-Fi and/or Zigbee), and be connected to the external electronic device (e.g., the external electronic device 320 of FIG. 3) through short-range wireless communication to receive data transmitted by the external electronic device 320 (e.g., the user's bio-signals acquired (or measured) by the external electronic device 320).

The processor 420 may be operatively connected to the communication circuit 410 to control the operation of the communication circuit 410.

The memory 430 may store instructions that may be executed by the processor 420. The operation of the processor 420 described below may be performed according to the execution of the instructions stored in the memory 430.

The processor 420 may receive a user's heart rate and/or heart rate variability measured by the external electronic device 320 from the external electronic device 320.

The external electronic device 320 may measure a heart rate and/or heart rate variability at each designated time (or cycle) in a state in which the external electronic device 320 is worn on a part (e.g., wrist and/or finger) of the user's body.

The heart rate may refer to the number of heart beats in a minute. The heart rate may change according to the user's state. According to an embodiment, in case the user performs intense exercise, the heart rate may have a relatively high value. Further, in case the user is in a stable state, the heart rate may have a relatively low value. In case the user is in a sleep state, the heart rate may have a lower value compared to other states.

The heart rate variability may be an indicator of changes in the time interval between heart beats. The heart rate variability may be a value related to the user's physical health. According to an embodiment, the user's body may be in a healthier state if the heart rate variability is high. Conversely, the user's body may be in a relatively unhealthy state if the heart rate variability is low.

A first sensor (e.g., a biometric sensor) included in the external electronic device 320 emits (or outputs) an optical signal to the user's body and receives and/or analyzes a signal reflected by the user's body; thus, the external electronic device 320 may measure (or acquire) the user's heart rate and/or heart rate variability.

Information including a heart rate and/or heart rate variability may be received by the communication circuit 410 through short-range wireless communication.

The processor 420 may receive the heart rate and/or heart rate variability transmitted by the external electronic device 320 and store the heart rate and/or the heart rate variability in the memory 430.

According to an embodiment, the processor 420 may store mapping data that maps the user's state that is determined based on the user's movement and/or heart rate and the heart rate and/or heart rate variability measured in the determined state in the memory 430.

The user's state may include the user's sleep state. Specifically, the processor 420 may map a heart rate and/or heart rate variability measured by the external electronic device 320 while the user is sleeping to the user's sleep state and store mapping data in the memory 430.

According to an embodiment, the user's sleep state may be distinguished according to the degree of movement of the sleeping user. For example, the user's sleep state may include a deep sleep state, which is a state in which the user's movement is lowest and in which the user's heart rate is relatively low, a REM sleep state, which is a state in which the user's movement is lowest and in which the user's heart rate is relatively high, a shallow sleep state, which is a state in which the degree of movement of the sleeping user is relatively high, and/or a state of being awakened during sleep, which is a state in which the degree of movement of the sleeping user is relatively high and in which the user's heart rate is relatively high. It is to be understood that the four sleep states described above are only examples that can vary from user to user and may be implemented in various ways by the user and/or the manufacturer of the electronic device 310.

The processor 420 may receive information indicating a level of movement of the user measured by the external electronic device 320 through short-range wireless communication and may distinguish (or determine, configure) sleep stages based on the user's level of movement.

According to an embodiment, in case the electronic device 310 is worn on at least a part of the user's body, the processor 420 may distinguish (or determine, or configure) sleep stages based on information indicating a level of movement of the user measured by the electronic device 310.

The processor 420 may distinguish a sleep state during the user's sleep time and store mapping data in which a heart rate and/or heart rate variability measured in each distinguished sleep state are/is mapped to the sleep state in the memory 430. Table 1 represents examples of mapping data.

**[Table 1]**

| Sleep state | Heart rate | Heart rate variability (e.g., standard deviation of the NN interval (SDNN) or square root of the mean of the sum of the square of differences between adjacent NN intervals (RMSSD)) |
|---|---|---|
| Deep sleep | 42bpm | 80ms |
| REM sleep | 52bpm | 70ms |
| Shallow sleep | 54bpm | 60ms |
| Wake up from sleep | 62bpm | 62ms |

The processor 420 may store mapping data in which a heart rate and/or heart rate variability are/is mapped to the sleep state in the memory 430, as in the example described above.

The processor 420 may determine any one of at least one heart rate measured while the user is sleeping as a sleep heart rate (sleep HRV) referring to a heart rate (or the most reliable heart rate) representing heart rates measured during sleep with reference to mapping data (or heart rate and/or heart rate variability).

Determining (or selecting) a sleep heart rate may be configured or provided for generating and/or providing accurate (or reliable) information related to the user's body based on a sleep heart rate representing the measured heart rates.

In selecting (or determining) a sleep heart rate among the measured heart rates, the processor 420 may refer to a sleep state included in the mapping data. According to an embodiment, the processor 420 may select a heart rate measured in a deep sleep state among the measured heart rates as a sleep heart rate. The deep sleep state is a state in which the user's body may recover the most, and a heart rate measured in a deep sleep state may be a heart rate representing the measured heart rates.

In case there are multiple heart rates measured in a deep sleep state, the processor 420 may determine (or select) an average of heart rates measured in a deep sleep state as a sleep heart rate.

The processor 420 may determine any one of a heart rate measured in a deep sleep state among the measured heart rates and a heart rate (or average of heart rates) measured from a time point before a designated time (e.g., 5 to 30 minutes) from the user's wake up time to the wake up time as a sleep heart rate.

In the case of a user with a specific pattern among users, a heart rate measured from a time point before a designated time from the wake-up time to the wake-up time than a heart rate measured in a deep sleep state may be a more reliable heart rate in representing the user's physical health. The specific pattern may include a pattern with a lot of activity amount before the user enters sleep. In the case of a user who has a lot of activity amount before a designated sleep time (e.g., 1-3 hours), a heart rate measured in a deep sleep state may be higher than a heart rate measured from a time point before a designated time from the wake-up time to the wake-up time, and a heart rate measured from a time point before a designated time from the wake-up time to the wake-up time may be a more reliable heart rate in representing the user's physical health.

The processor 420 may compare a heart rate measured in a deep sleep state among the measured heart rates and a heart rate (average of heart rates) measured from a time point before a designated time (e.g., 5 to 30 minutes) from the user's wake up time to the wake up time and determine (or select) a heart rate with a lower magnitude as a sleep heart rate.

According to an embodiment, the processor 420 may determine (or select) a heart rate (or average of heart rates) measured from a time point before a designated time (e.g., 5 to 30 minutes) from the user's wake up time to the wake up time as a sleep heart rate. The processor 420 may monitor (or identify) the user's activity amount before a designated time for the user to go to bed, and in case the user's activity amount satisfies a designated condition (e.g., a condition in which the user's activity amount is greater than or equal to a designated magnitude), the processor 420 may determine (or select) a heart rate (or average of heart rates) measured from a time point before a designated time (e.g., 5 to 30 minutes) from the user's wake up time to the wake up time as a sleep heart rate. In case the user's activity amount satisfies a designated condition (e.g., a condition in which the user's activity amount is greater than or equal to a designated magnitude), the processor 420 may not identify the user's heart rate during sleep.

The processor 420 may determine any one of at least one heart rate variability measured while the use is sleeping as sleep heart rate variability (sleep HRV) referring to heart rate variability (or the most reliable heart rate variability) representing heart rate variability measured during sleep with reference to mapping data (or a heart rate and/or heart rate variability).

Determining (or selecting) sleep heart rate variability may be for generating and/or providing accurate (or reliable) information related to the user's body based on the sleep heart rate variability representing the measured heart rate variability.

In selecting (or determining) sleep heart rate variability among the measured heart rate variability, the processor 420 may refer to a sleep state included in mapping data. According to an embodiment, the processor 420 may select heart rate variability measured in a deep sleep state among the measured heart rate variability as sleep heart rate variability. The deep sleep state is a state in which the user's body may recover the most, and heart rate variability measured in a deep sleep state may be heart rate variability representing the measured heart rate variability.

In case there are multiple heart rate variabilities measured in a deep sleep state, the processor 420 may determine (or select) an average of heart rate variabilities measured in a deep sleep state as a sleep heart rate variability.

The processor 420 may determine any one of heart rate variability measured in a deep sleep state among the measured heart rate variability and heart rate variability (or average of heart rate variability) measured from a time point before a designated time (e.g., 5 to 30 minutes) from the user's wake up time to the wake up time as sleep heart rate variability.

In the case of a user with a specific pattern among users, heart rate variability measured from a time point before a designated time from the wake-up time to the wake-up time may be more reliable than heart rate variability measured in a deep sleep state in representing the user's physical health. The specific pattern may include a pattern with a lot of activity amount before the user enters sleep. In the case of a user who has a lot of activity amount before a designated sleep time (e.g., 1-3 hours), heart rate variability measured in a deep sleep state may be lower than heart rate variability measured from a time point before a designated time from the wake-up time to the wake-up time, and heart rate variability measured from a time point before a designated time from the wake-up time to the wake-up time may be a more reliable heart rate variability in representing the user's physical health.

The processor 420 may compare heart rate variability measured in a deep sleep state among the measured heart rate variability and heart rate variability (or average of heart rate variability) measured from a time point before a designated time (e.g., 5 to 30 minutes) from the user's wake up time to the wake up time and determine (or select) heart rate variability with a larger magnitude as sleep heart rate variability.

According to an embodiment, the processor 420 may determine (or select) heart rate variability (or average of heart rate variability) measured from a time point before a designated time (e.g., 5 to 30 minutes) from the user's wake up time to the wake up time as sleep heart rate variability. The processor 420 may monitor (or identify) the user's activity amount before a designated time for the user to go to bed, and in case the user's activity amount satisfies a designated condition (e.g., a condition in which the user's activity amount is greater than or equal to a designated magnitude), the processor 420 may determine (or select) heart rate variability (or average of heart rate variability) measured from a time point before a designated time (e.g., 5 to 30 minutes) from the user's wake up time to the wake up time as sleep heart rate variability. In case the user's activity amount satisfies a designated condition (e.g., a condition in which the user's activity amount is greater than or equal to a designated magnitude), the processor 420 may not identify the user's heart rate variability during sleep.

The processor 420 may perform at least one operation based on a sleep heart rate and/or sleep heart rate variability.

At least one operation based on the sleep heart rate and/or sleep heart rate variability may include an operation of displaying (or outputting) the sleep heart rate and/or sleep heart rate variability.

According to an embodiment, the processor 420 may control to display a sleep heart rate and/or sleep heart rate variability on the display (e.g., the display module 160 of FIG. 1).

The sleep heart rate displayed on the display 160 may be displayed as in a sleep heart rate according to the user's sleep time and be displayed along with the history of the sleep heart rate.

The sleep heart rate variability displayed on the display 160 may be displayed as in sleep heart rate variability according to the user's sleep time and be displayed along with the history of sleep heart rate variability.

The sleep heart rate and/or sleep heart rate variability may be displayed on the display 160 as in a sleep heart rate and/or sleep heart rate variability of a user other than the user.

According to an embodiment, the processor 420 may provide a user interface that enables the user to input a sleep heart rate and/or sleep heart rate variability.

At least one operation based on the sleep heart rate and/or sleep heart rate variability may include an operation of providing information related to the user's vitality state determined (or generated) based on the sleep heart rate and/or sleep heart rate variability.

Information related to the user's vitality state may include a score that quantifies the user's vitality state. The score that quantifies a user's vitality state may be referred to as a vitality score. A higher user's vitality score may indicate a state that the user may perform a higher intensity of activity. Conversely, a lower user's vitality score may indicate a state that the user should avoid a higher intensity of activity. For example, the processor 420 may identify a sleep heart rate and/or sleep heart rate variability and determine (or calculate) the user's vitality score based on the sleep heart rate and/or sleep heart rate variability. According to an embodiment, the processor 420 may produce (or output, determine, calculate) a higher vitality score as the sleep heart rate is lower and/or the sleep heart rate variability is higher. The processor 420 may produce (or output, determine, calculate) a lower vitality score as the sleep heart rate is higher and/or the sleep heart rate variability is lower. The processor 420 may determine a vitality score in consideration of the user's various information (e.g., the user's exercise history, the user's sleep time) as well as the sleep heart rate and/or sleep heart rate variability. For example, in case the user's sleep time is similar to the user's average sleep time, the processor 420 may produce (or output, determine, calculate) a higher vitality score. In case the user's exercise time is higher than the user's average exercise time, the processor 420 may produce (or output, determine, calculate) a lower vitality score. The processor 420 may control to display the determined vitality score on the display 160 or provide the determined vitality score to the user in various ways.

Information related to the user's vitality state may include information that may be used as a reference for the user to perform an activity after waking up. For example, information related to the user's vitality state may include information that guides the user to perform an appropriate intensity of activity after waking up. For example, the processor 420 may identify a sleep heart rate and/or sleep heart rate variability and determine whether the user's vitality state is sufficient to perform a high intensity of activity. The processor 420 may identify that the user's vitality state is sufficient to perform a high intensity of activity and provide a suggestion to the user to perform a high intensity of activity in various forms (e.g., screen displayed on the display 160, sound output through the speaker). The processor 420 may identify that the user's vitality state is difficult to perform a high intensity of activity and provide a suggestion to the user to perform a low intensity of activity in various forms (e.g., screen displayed on the display 160, sound output through the speaker).

Information related to the user's vitality state may include guide information that enables the user to manage a heart rate and/or heart rate variability. The processor 420 may display guide information related to a sleep time on the display 160. Alternatively, in case of a high heart rate and/or low heart rate variability, the processor 420 may display guide information that may guide activities that may reduce a user's stress (e.g., yoga, meditation, deep breathing activities, activities that enable progressive muscle relaxation) on the display 160. Alternatively, in case of a high heart rate and/or low heart rate variability, the processor 420 may display guide information that may guide activities that may lower a heart rate (e.g., regular low intensity exercise) on the display 160. In case of a high heart rate and/or low heart rate variability, the processor 420 may display guidance information on lifestyle habits that may lower a heart rate (e.g., diet information that may lower a heart rate, diet information that increases a heart rate) on the display 160.

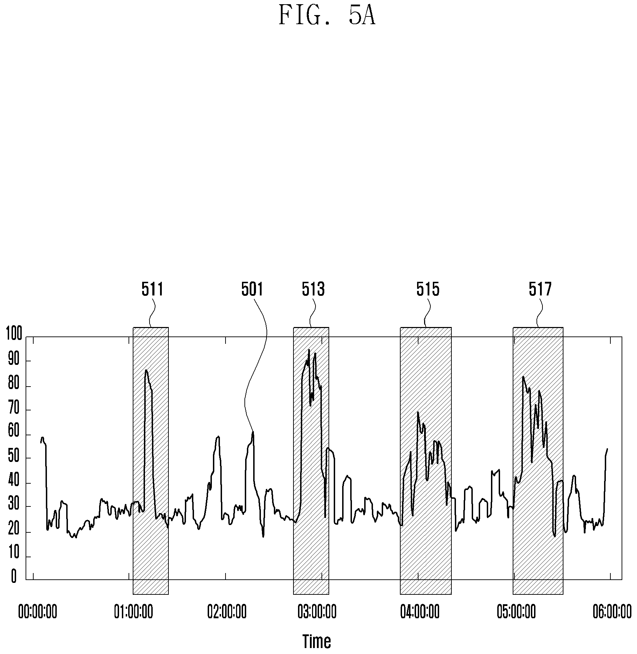

FIG. 5A is a graph illustrating heart rate variability according to sleep stages in an electronic device according to an embodiment.

FIG. 5A illustrates the user's heart rate variability (HRV) 501 in which an external electronic device (e.g., the external electronic device 320 of FIG. 3) measures in the user's sleep state.

The heart rate variability may be an indicator representing changes in a time interval between heart beats. The heart rate variability may be a value related to the user's physical health. According to an embodiment, the user's body may be in a healthier state if the heart rate variability is high. Conversely, the user's body may be in a relatively unhealthy state if the heart rate variability is low.

With reference to FIG. 5A, it may be identified that the heart rate variability 501 has a higher value when the user's sleep state is in deep sleep states 511, 513, 515, and 517 compared to other states (e.g., a shallow sleep state, an REM sleep state, a state of being awakened from sleep). Heart rate variability measured in other states may be similar to heart rate variability measured in a state in which the user is active. Therefore, heart rate variability measured in a deep sleep state may be heart rate variability that may represent a sleep state. The processor (e.g., the processor 420 of FIG. 4) may determine heart rate variability measured in a deep sleep state as sleep heart rate variability.

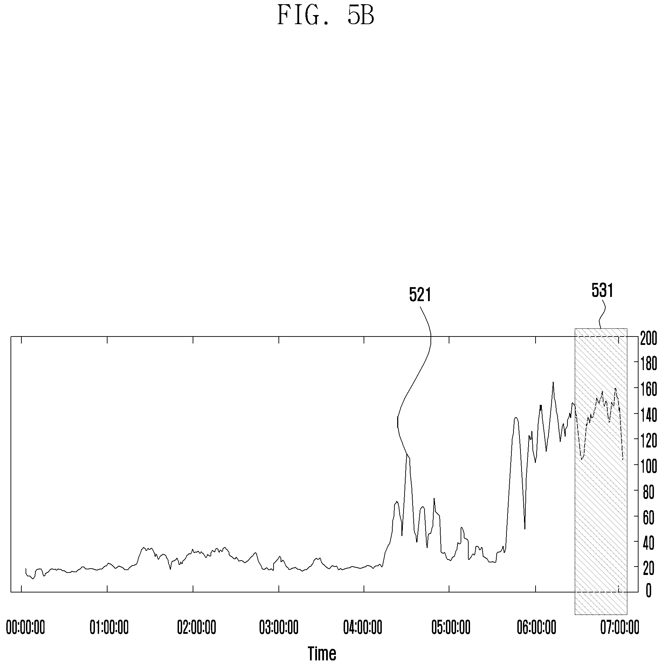

FIG. 5B is a graph illustrating sequential heart rate variability in an electronic device according to an embodiment.

FIG. 5B illustrates the user's heart rate variability (HRV) 521 in which an external electronic device (e.g., the external electronic device 320 of FIG. 3) measures in the user's sleep state.

With reference to the heart rate variability 521 illustrated in FIG. 5B, heart rate variability 521 measured from a time point before a designated time (e.g., 5 to 30 minutes) from the wake-up time to a wake-up time 531 may be higher than heart rate variability measured at other times.

A user measured with the heart rate variability 521 illustrated in FIG. 5B may be a user with a specific behavioral pattern. The specific behavioral pattern may include a behavioral pattern having a lot of activity amount before the user enters sleep. In the case of a user with a specific behavioral pattern, a heart rate measured from a time point before a designated time from the wake-up time to the wake-up time than a heart rate measured in a deep sleep state may be a more reliable heart rate in representing the user's physical health.

In the case of a user who has a lot of activity amount before a designated sleep time (e.g., 1-3 hours), a heart rate measured in a deep sleep state may be higher than a heart rate measured from a time point before a designated time from the wake-up time to the wake-up time, and a heart rate measured from a time point before a designated time from the wake-up time to the wake-up time may be a more reliable heart rate in representing the user's physical health.

Therefore, the processor 420 may compare heart rate variability measured in a deep sleep state among the measured heart rate variability and heart rate variability (or average of heart rate variability) measured from a time point before a designated time (e.g., 5 to 30 minutes) from the user's wake up time to the wake up time and determine (or select) heart rate variability with a larger magnitude as sleep heart rate variability.

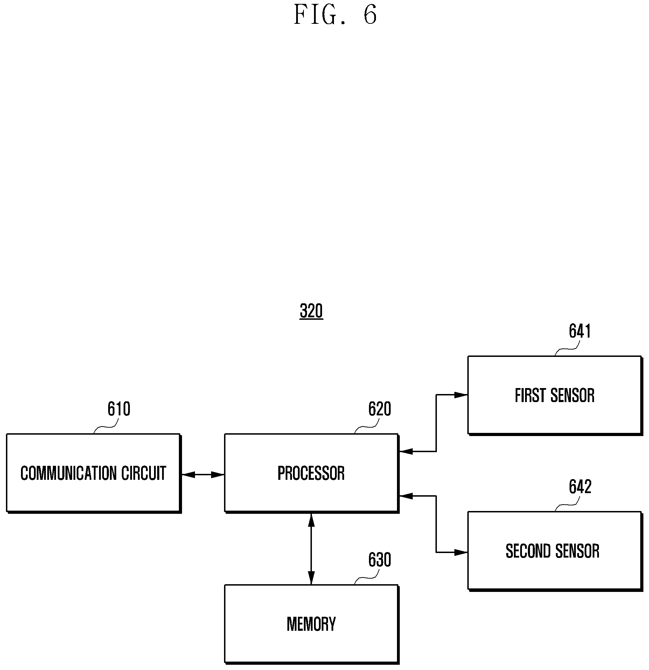

FIG. 6 is a block diagram illustrating an external electronic device according to an embodiment.

According to an embodiment, the external electronic device (e.g., the electronic device 320 of FIG. 3) may include a communication circuit 610 (e.g., the wireless communication module 192 of FIG. 1), a processor 620 (e.g., the processor 120 of FIG. 1), a memory 630 (e.g., the memory 130 of FIG. 1), a first sensor 641 (e.g., the sensor module 176 of FIG. 1), and/or a second sensor 642 (e.g., the sensor module 176 of FIG. 1). The external electronic device 320 can include at least one or more additional sensors in additional to the first sensor 641 and the second sensor 642.

The communication circuit 610 may include various circuit structures used for modulating and/or demodulating signals within the external electronic device 320. For example, the communication circuit 610 may modulate a baseband signal into a radio frequency (RF) band signal so as to output through an antenna (not illustrated) or may demodulate an RF band signal received through an antenna into a baseband signal and transmit the baseband signal to the processor 620. The communication circuit 610 may support short-range wireless communication (e.g., Bluetooth, low-power Bluetooth, Wi-Fi and/or Zigbee), and be connected to the electronic device (e.g., the electronic device 310) through short-range wireless communication to transmit the user's biometric information acquired (or measured) by the external electronic device 320 to the electronic device.

The processor 620 may be operatively connected to the communication circuit 610, the first sensor 641, and/or the second sensor 642 to control the operation of the communication circuit 610, the first sensor 641 and/or the second sensor 642.

The memory 630 may store instructions that may be executed by the processor 620. The operation of the processor 620 described below may be performed according to the execution of the instructions stored in the memory 630.

The first sensor 641 may measure (or sense, detect) the user's biometric information in a state in which the external electronic device 320 is worn on a part (e.g., the user's wrist, the user's finger) of the user's body.

For example, the user's biometric information may include information related to the user's health. For example, the user's biometric information may include at least one of the user's heart rate (HR), heart rate variability, electrocardiogram (ECG), blood pressure, or oxygen saturation.

The heart rate may refer to the number of heart beats in a minute. The heart rate variability may be an indicator of changes in the time interval between heart beats. The electrocardiogram may refer to an electrical signal of the heart according to the contraction and/or relaxation of the heart. The blood pressure may refer to a pressure applied to blood vessels due to the movement of blood. The oxygen saturation may refer to the extent to which hemoglobin in the blood includes oxygen.

According to an embodiment, the first sensor 641 may include at least one of a biometric optical sensor or an electrocardiogram sensor.

The first sensor 641 may include a light source that outputs a signal (or light having a wavelength with a designated magnitude) to a part of the user's body in order to acquire the user's biometric information.

The light source may include at least one of a light source capable of outputting green light for measuring a heart rate and/or heart rate variability, a light source capable of outputting red light capable of measuring a more accurate heart rate and/or heart rate variability, a light source capable of outputting infrared rays capable of measuring oxygen saturation, or a blue light source capable of measuring blood sugar levels. The processor 620 may acquire the user's biometric information using a combination of signals (or light) acquired by at least one light source.

The second sensor 642 may measure (or sense, detect) a movement of the user's body in a state in which the external electronic device 320 is worn on a part of the user's body. According to an embodiment, the second sensor 642 may include at least one sensor of an acceleration sensor capable of measuring the speed and/or acceleration of the external electronic device 320 and/or a gyro sensor capable of measuring the posture of the external electronic device 320.

The processor 620 may receive information related to the user's movement from the second sensor 642 and identify the user's state. The user's state may include a sleep state in which the user enters sleep or an activity state in which the user is active.

The processor 620 may identify whether the user's state is in a sleep state using the second sensor 642. According to an embodiment, the processor 620 may identify whether the user is moving based on information related to the user's movement collected using the second sensor, and in case the frequency of occurrence of the user's movement and/or the magnitude of the user's movement are/is a designated value or less, the processor 620 may determine that the user's state is in a sleep state.

Alternatively, the processor 620 may receive information related to the user's movement from the second sensor 642 and transmit information related to the user's movement to the electronic device 310. The electronic device 310 may identify whether the user's state is in a sleep state based on information related to the user's movement. The electronic device 310 may identify whether the user is moving based on information related to the user's movement, and in case the frequency of occurrence of the user's movement and/or the magnitude of the user's movement are/is a designated value or less, the electronic device 310 may determine that the user's state is in a sleep state.

The processor 620 may control the first sensor 641 to acquire the user's biometric information in a sleep state.

According to an embodiment, the processor 620 may control the first sensor 641 to emit (or output) an optical signal to the user's body at designated intervals and to receive a signal reflected by the user's body. The processor 620 may analyze the reflected signal and determine (or identify, acquire) a heart rate and/or heart rate variability based on the analysis results.

The processor 620 may control the communication circuit 610 to transmit data including the determined heart rate and/or heart rate variability to the electronic device 310.

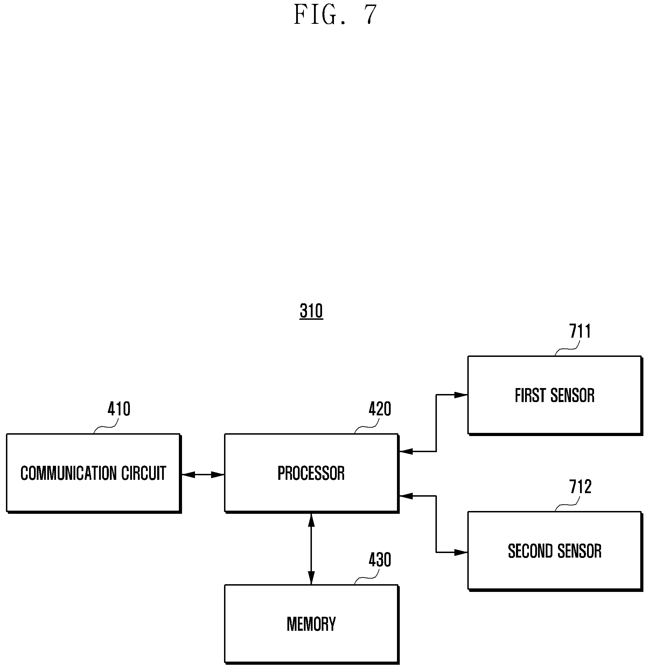

FIG. 7 is a block diagram illustrating an electronic device according to an embodiment.

According to an embodiment, the electronic device (e.g., the electronic device 310 of FIG. 3) may include a communication circuit 410 (e.g., the wireless communication module 192 of FIG. 1), a processor 420 (e.g., the processor 120 of FIG. 1), a memory 430 (e.g., the memory 130 of FIG. 1), a first sensor 711 (e.g., the sensor module 176 of FIG. 1), and a second sensor 712 (e.g., the sensor module 176 of FIG. 1). The electronic device 310 can include at least one or more additional sensors in additional to the first sensor 711 and the second sensor 712.

The communication circuit 410 may include various circuit structures used for modulating and/or demodulating signals within the electronic device 310. For example, the communication circuit 410 may modulate a baseband signal into a radio frequency (RF) band signal so as to output through an antenna (not illustrated) or may demodulate an RF band signal received through an antenna into a baseband signal and transmit the baseband signal to the processor 420. The communication circuit 410 may support short-range wireless communication (e.g., Bluetooth, low-power Bluetooth, Wi-Fi and/or Zigbee). The communication circuit 410 may transmit the user's biometric information (e.g., heart rate and/or heart rate variability) measured by the electronic device 310 to the external device (e.g., a server that may manage the user's biometric information).

The first sensor 711 may measure (or sense, detect) the user's biometric information in a state in which the electronic device 310 is worn on a part of the user's body.

For example, the user's biometric information may include information related to the user's health. For example, the user's biometric information may include at least one of the user's heart rate (HR), heart rate variability, electrocardiogram (ECG), blood pressure, or oxygen saturation.

According to an embodiment, the first sensor 711 may include at least one of a biometric optical sensor or an electrocardiogram sensor.

The first sensor 711 may include a light source that outputs a signal (or light having a wavelength with a designated magnitude) to a part of the user's body in order to acquire the user's biometric information.

The light source may include at least one of a light source capable of outputting green light for measuring a heart rate and/or heart rate variability, a light source capable of outputting red light capable of measuring a more accurate heart rate and/or heart rate variability, a light source capable of outputting infrared rays capable of measuring oxygen saturation, or a blue light source capable of measuring blood sugar levels.

The second sensor 712 may measure (or sense, detect) movements of the user's body in a state in which the electronic device 310 is worn on a part of the user's body. According to an embodiment, the second sensor 712 may include at least one sensor of an acceleration sensor capable of measuring the speed and/or acceleration of the electronic device 310 and/or a gyro sensor capable of measuring the posture of the electronic device 310.

The processor 420 may be operatively connected to the communication circuit 410 to control the operation of the communication circuit 410.

The memory 430 may store instructions that may be executed by the processor 420. The operation of the processor 420 described below may be performed according to the execution of the instructions stored in the memory 430.

The processor 420 may measure the user's heart rate and/or heart rate variability using the first sensor 711.

The processor 420 may receive information related to the user's movement from the second sensor 712 and identify the user's state. The user's state may include a sleep state in which the user enters sleep or an activity state in which the user is active.

The processor 420 may identify whether the user's state is in a sleep state using the second sensor 712. According to an embodiment, the processor 420 may identify whether the user is moving based on information related to the user's movement collected using the second sensor, and in case the frequency of occurrence of the user's movements and/or the magnitude of the user's movements are/is a designated value or less, the processor 420 may determine that the user's state is in a sleep state.

The processor 420 may control the first sensor 711 to acquire the user's biometric information in a sleep state.

According to an embodiment, the processor 420 may control the first sensor 711 to emit (or output) an optical signal to the user's body at designated intervals and to receive a signal reflected by the user's body. The processor 420 may analyze the reflected signal and determine (or identify, acquire) a heart rate and/or heart rate variability based on the analysis results.

The heart rate may refer to the number of heart beats in a minute. The heart rate may change according to the user's state. According to an embodiment, in case the user performs intense exercise, the heart rate may have a relatively high value. Further, in case the user is in a stable state, the heart rate may have a relatively low value. In case the user is in a sleep state, the heart rate may have a lower value compared to other states.

The heart rate variability may be an indicator of changes in the time interval between heart beats. The heart rate variability may be a value related to the user's physical health. According to an embodiment, the user's body may be in a healthier state if the heart rate variability is high. Conversely, the user's body may be in a relatively unhealthy state if the heart rate variability is low.

The processor 420 may emit (or output) an optical signal to the user's body and receive and/or analyze a signal reflected by the user's body; thus, the processor 420 may control the first sensor 711 to measure (or acquire) the user's heart rate and/or heart rate variability.

The processor 420 may receive a heart rate and/or heart rate variability and store the heart rate and/or the heart rate variability in the memory 430.

According to an embodiment, the processor 420 may store mapping data that maps the user's state determined based on the user's movement and/or heart rate and the heart rate and/or heart rate variability measured in the determined state in the memory 430.

The user's state may include the user's sleep state. Specifically, the processor 420 may map a heart rate and/or heart rate variability measured by the external electronic device 320 while the user is sleeping to the user's sleep state and store mapping data in the memory 430.

According to an embodiment, the user's sleep state may be distinguished according to the degree of movement of the sleeping user. For example, the user's sleep state may include a deep sleep state, which is a state in which the user's movement is lowest and in which the user's heart rate is relatively low, a REM sleep state, which is a state in which the user's movement is lowest and in which the user's heart rate is relatively high, a shallow sleep state, which is a state in which the degree of movement of the sleeping user is relatively high, and/or a state of being awakened during sleep, which is a state in which the degree of movement of the sleeping user is relatively high and in which the user's heart rate is relatively high. Four sleep states described above are only examples and may be implemented in various ways by the user and/or the manufacturer of the electronic device 310.

The processor 420 may distinguish (or determine, configure) sleep stages based on the user's level of movement.

According to an embodiment, in case the electronic device 310 or the external electronic device 320 is worn on at least a part of the user's body, the processor 420 may distinguish (or determine, configure) sleep stages based on information indicating a level of movement of the user measured by the second sensor 712.

The processor 420 may distinguish a sleep state during the user's sleep time and store mapping data in which a heart rate and/or heart rate variability measured in each distinguished sleep state are/is mapped to the sleep state in the memory 430.

The processor 420 may store mapping data in which a heart rate and/or heart rate variability are/is mapped to sleep states in the memory 430.

The processor 420 may determine any one of at least one heart rate measured while the user is sleeping as a sleep heart rate (sleep HR) indicating a representative heart rate (or the most reliable heart rate) of heart rates measured during sleep with reference to mapping data (or heart rate and/or heart rate variability).

Determining (or selecting) a sleep heart rate may be for generating and/or providing accurate (or reliable) information related to the user's body based on a sleep heart rate representing the measured heart rates.

In selecting (or determining) a sleep heart rate among the measured heart rates, the processor 420 may refer to the sleep state included in mapping data. According to an embodiment, the processor 420 may select a heart rate measured in a deep sleep state among the measured heart rates as a sleep heart rate. The deep sleep state is a state in which the user's body may recover the most, and a heart rate measured in a deep sleep state may be a heart rate representing the measured heart rates.

In case there are multiple heart rates measured in a deep sleep state, the processor 420 may determine (or select) average of heart rates measured in a deep sleep state as a sleep heart rate.

The processor 420 may determine any one of a heart rate measured in a deep sleep state among the measured heart rates and a heart rate (or average of heart rates) measured from a time point before a designated time (e.g., 5 to 30 minutes) from the user's wake up time to the wake up time as a sleep heart rate.

In the case of a user with a specific pattern among users, a heart rate measured from a time point before a designated time from the wake-up time to the wake-up time than a heart rate measured in a deep sleep state may be a more reliable heart rate in representing the user's physical health. The specific pattern may include a pattern with a lot of activity amount before the user enters sleep. In the case of a user who has a lot of activity amount before a designated sleep time (e.g., 1-3 hours), a heart rate measured in a deep sleep state may be higher than a heart rate measured from a time point before a designated time from the wake-up time to the wake-up time, and a heart rate measured from a time point before a designated time from the wake-up time to the wake-up time may be a more reliable heart rate in representing the user's physical health.

The processor 420 may compare a heart rate measured in a deep sleep state among the measured heart rates and a heart rate (average of heart rates) measured from a time point before a designated time (e.g., 5 to 30 minutes) from the user's wake up time to the wake up time and determine (or select) a heart rate with a lower magnitude as a sleep heart rate.

According to an embodiment, the processor 420 may determine (or select) a heart rate (or average of heart rates) measured from a time point before a designated time (e.g., 5 to 30 minutes) from the user's wake up time to the wake up time as a sleep heart rate. The processor 420 may monitor (or identify) the user's activity amount before a designated time for the user to go to bed, and in case the user's activity amount satisfies a designated condition (e.g., a condition in which the user's activity amount is greater than or equal to a designated magnitude), the processor 420 may determine (or select) a heart rate (or average of heart rates) measured from a time point before a designated time (e.g., 5 to 30 minutes) from the user's wake up time to the wake up time as a sleep heart rate. In case the user's activity amount satisfies a designated condition (e.g., a condition in which the user's activity amount is greater than or equal to a designated magnitude), the processor 420 may not identify the user's heart rate during sleep.

The processor 420 may determine any one of at least one heart rate variability measured while the use is sleeping as sleep heart rate variability (sleep HRV) referring to heart rate variability (or the most reliable heart rate variability) representing heart rate variability measured during sleep with reference to mapping data (or heart rate and/or heart rate variability).

Determining (or selecting) sleep heart rate variability may be for generating and/or providing accurate (or reliable) information related to the user's body based on sleep heart rate variability representing the measured heart rate variability.

In selecting (or determining) sleep heart rate variability among the measured heart rate variability, the processor 420 may refer to a sleep state included in the mapping data. According to an embodiment, the processor 420 may select heart rate variability measured in a deep sleep state among the measured heart rate variability as sleep heart rate variability. The deep sleep state is a state in which the user's body may recover the most, and heart rate variability measured in a deep sleep state may be heart rate variability representing the measured heart rate variability.

In case there are multiple heart rates measured in a deep sleep state, the processor 420 may determine (or select) an average of heart rate variability measured in a deep sleep state as sleep heart rate variability.

The processor 420 may determine any one of heart rate variability measured in a deep sleep state among the measured heart rate variability and heart rate variability (or average of heart rate variability) measured from a time point before a designated time (e.g., 5 to 30 minutes) from the user's wake up time to the wake up time as sleep heart rate variability.

In case a user has a specific pattern among users, heart rate variability measured from a time point before a designated time from the wake-up time to the wake-up time than heart rate variability measured in a deep sleep state may be more reliable heart rate variability in representing the user's physical health. The specific pattern may include a pattern with a lot of activity amount before the user enters sleep. In the case of a user who has a lot of activity amount before a designated sleep time (e.g., 1-3 hours), heart rate variability measured in a deep sleep state may be lower than heart rate variability measured from a time point before a designated time from the wake-up time to the wake-up time, and heart rate variability measured from a time point before a designated time from the wake-up time to the wake-up time may be more reliable heart rate variability in representing the user's physical health.

The processor 420 may compare heart rate variability measured in a deep sleep state among the measured heart rate variability and heart rate variability (or average of heart rate variability) measured from a time point before a designated time (e.g., 5 to 30 minutes) from the user's wake up time to the wake up time and determine (or select) heart rate variability with a larger magnitude as sleep heart rate variability.

According to an embodiment, the processor 420 may determine (or select) heart rate variability (or average of heart rate variability) measured from a time point before a designated time (e.g., 5 to 30 minutes) from the user's wake up time to the wake up time as sleep heart rate variability. The processor 420 may monitor (or identify) the user's activity amount before a designated time for the user to go to bed, and in case the user's activity amount satisfies a designated condition (e.g., a condition in which the user's activity amount is greater than or equal to a designated magnitude), the processor 420 may determine (or select) heart rate variability (or average of heart rate variability) measured from a time point before a designated time (e.g., 5 to 30 minutes) from the user's wake up time to the wake up time as sleep heart rate variability. In case the user's activity amount satisfies a designated condition (e.g., a condition in which the user's activity amount is greater than or equal to a designated magnitude), the processor 420 may not identify the user's heart rate variability during sleep.

The processor 420 may perform at least one operation based on the sleep heart rate and/or sleep heart rate variability.

At least one operation based on the sleep heart rate and/or sleep heart rate variability may include an operation of displaying (or outputting) the sleep heart rate and/or sleep heart rate variability.

According to an embodiment, the processor 420 may control to display a sleep heart rate and/or sleep heart rate variability on the display (e.g., the display module 160 of FIG. 1).

The sleep heart rate displayed on the display 160 may be displayed as in a sleep heart rate according to the user's sleep time and be displayed along with the history of the sleep heart rate.

The sleep heart rate variability displayed on the display 160 may be displayed as in sleep heart rate variability according to the user's sleep time and be displayed along with the history of sleep heart rate variability.

The sleep heart rate and/or sleep heart rate variability may be displayed on the display 160 as in a sleep heart rate and/or sleep heart rate variability of a user other than the user.

According to an embodiment, the processor 420 may provide a user interface that enables the user to input a sleep heart rate and/or sleep heart rate variability.

At least one operation based on the sleep heart rate and/or sleep heart rate variability may include an operation of providing information related to the user's vitality state determined (or generated) based on the sleep heart rate and/or sleep heart rate variability.

Information related to the user's vitality state may include a score that quantifies the user's vitality state. The score that quantifies a user's vitality state may be referred to as a vitality score. A higher user's vitality score may indicate a state that the user may perform higher intensity of activity. Conversely, a lower user's vitality score may indicate a state that the user should avoid higher intensity of activity. For example, the processor 420 may identify a sleep heart rate and/or sleep heart rate variability and determine (or calculate) the user's vitality score based on the sleep heart rate and/or sleep heart rate variability. According to an embodiment, the processor 420 may produce (or output, determine, calculate) a higher vitality score as the sleep heart rate is lower and/or the sleep heart rate variability is higher. The processor 420 may produce (or output, determine, calculate) a lower vitality score as the sleep heart rate is higher and/or the sleep heart rate variability is lower. The processor 420 may determine a vitality score in consideration of the user's various information (e.g., the user's exercise history, the user's sleep time) as well as the sleep heart rate and/or sleep heart rate variability. For example, in case the user's sleep time is similar to the user's average sleep time, the processor 420 may produce (or output, determine, calculate) a higher vitality score. In case the user's exercise time is higher than the user's average exercise time, the processor 420 may produce (or output, determine, calculate) a lower vitality score. The processor 420 may control to display the determined vitality score on the display 160 or provide the determined vitality score to the user in various ways.

Information related to the user's vitality state may include information that may be used as a reference for the user to perform activity after waking up. For example, information related to the user's vitality state may include information that guides the user to perform appropriate intensity of activity after waking up. For example, the processor 420 may identify a sleep heart rate and/or sleep heart rate variability and determine whether the user's vitality state is sufficient to perform high intensity of activity. The processor 420 may identify that the user's vitality state is sufficient to perform high intensity of activity and provide a suggestion to the user to perform high intensity of activity in various forms (e.g., screen displayed on the display 160, sound output through the speaker). The processor 420 may identify that the user's vitality state is difficult to perform high intensity of activity and provide a suggestion to the user to perform low intensity of activity in various forms (e.g., screen displayed on the display 160, sound output through the speaker).

Information related to the user's vitality state may include guide information that enables the user to manage a heart rate and/or heart rate variability. The processor 420 may control to display guide information related to a sleep time on the display 160. Alternatively, in the case of a high heart rate and/or low heart rate variability, the processor 420 may control to display guide information that may guide activities that may reduce a user stress (e.g., yoga, meditation, deep breathing activities, activities that enable progressive muscle relaxation) on the display 160. Alternatively, in the case of a high heart rate and/or low heart rate variability, the processor 420 may control to display guide information that may guide activities that may lower a heart rate (e.g., regular low intensity exercise) on the display 160. In the case of a high heart rate and/or low heart rate variability, the processor 420 may control to display guidance information on lifestyle habits that may lower a heart rate (e.g., diet information that may lower a heart rate, diet information that increases a heart rate) on the display 160.

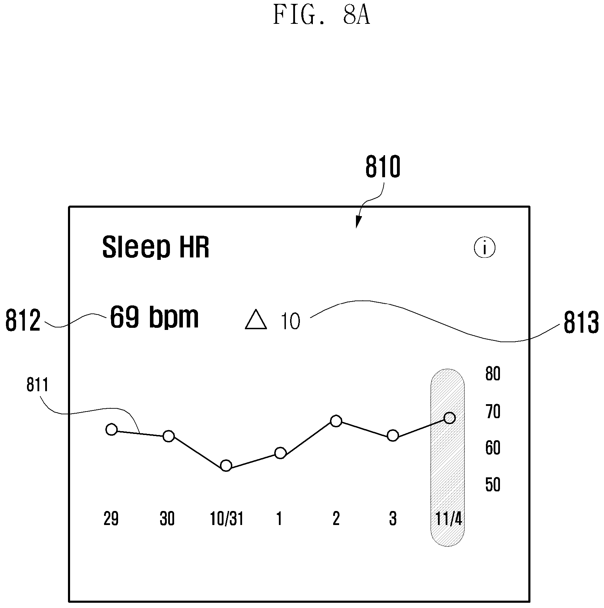

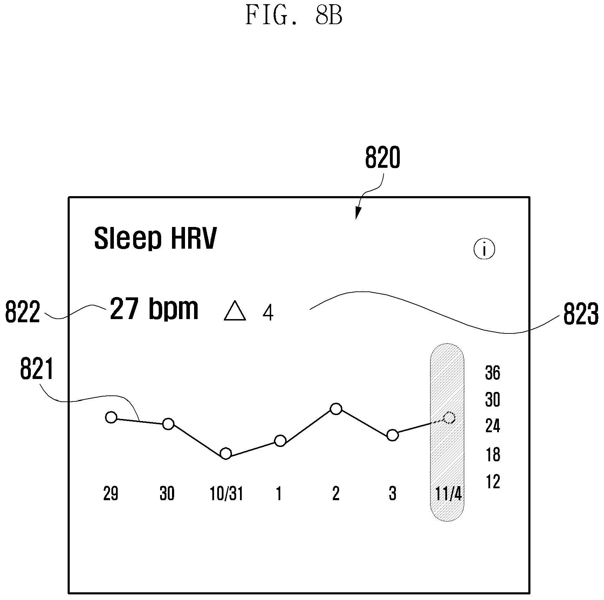

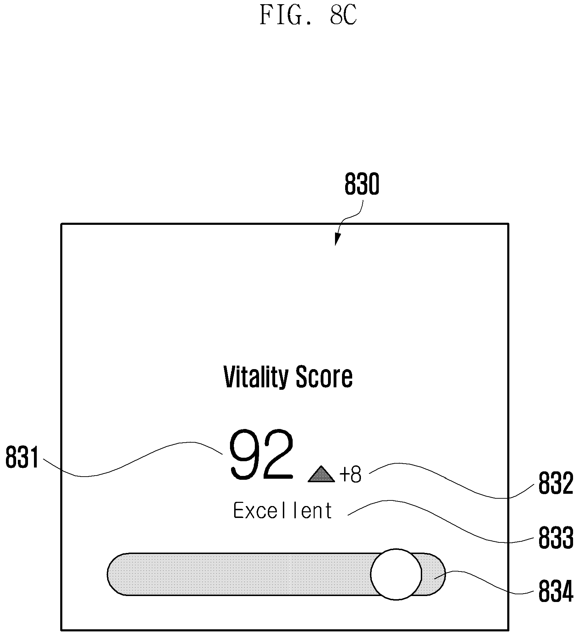

FIGS. 8A, 8B, and/or 8C are diagrams illustrating an electronic device performing at least one operation based on a sleep heart rate and/or sleep heart rate variability according to an embodiment.

According to an embodiment, the electronic device (e.g., the electronic device 310 of FIG. 4) may determine (or select) a sleep heart rate and/or sleep heart rate variability and perform at least one operation based on the sleep heart rate and/or sleep heart rate variability.

At least one operation based on the sleep heart rate and/or sleep heart rate variability may include an operation of displaying (or outputting) the sleep heart rate and/or sleep heart rate variability.

According to an embodiment, the electronic device 310 may display a sleep heart rate and/or sleep heart rate variability on the display (e.g., the display module 160 of FIG. 1).

With reference to FIG. 8A, the electronic device 310 may display a screen 810 including information related to a sleep heart rate on the display 160.

The screen 810 including information related to a sleep heart rate may include a graph 811 indicating the user's sleep heart rate determined during a designated time period (e.g., 1 week), a sleep heart rate (e.g., 69 BPM) 812 determined while the user was in the most recent sleep state, and/or information 813 indicating a difference value between the sleep heart rate (e.g., 69 BPM) 812 determined while the user was in the most recent sleep state and an average sleep heart rate.

The electronic device 310 may provide a screen 810 including information related to a sleep heart rate to the user, and induce the user to perform operations of identifying a sleep heart rate and/or regulating a sleep heart rate with reference to the screen 810 including information related to the sleep heart rate.

With reference to FIG. 8B, the electronic device 310 may display a screen 820 including information related to sleep heart rate variability on the display 160.

The screen 820 including information related to sleep heart rate variability may include a graph 821 indicating the user's sleep heart rate variability determined during a designated time period (e.g., 1 week), sleep heart rate variability (e.g., 27 ms) 822 determined while the user was in the most recent sleep state, and/or information 823 indicating a difference value between the sleep heart rate (e.g., 27 ms) 822 determined while the user was in the most recent sleep state and an average sleep heart rate.

The electronic device 310 may provide a screen 820 including information related to sleep heart rate variability to the user and induce the user to perform operations of identifying sleep heart rate variability and/or regulating sleep heart rate variability with reference to the screen 820 including information related to sleep heart rate variability.

At least one operation based on the sleep heart rate and/or sleep heart rate variability may include an operation of providing information related to the user's vitality state determined (or generated) based on the sleep heart rate and/or sleep heart rate variability.

Information related to the user's vitality state may include a score that quantifies the user's vitality state. The score that quantifies a user's vitality state may be referred to as a vitality score. A higher user's vitality score may indicate a state that the user may perform higher intensity of activity. Conversely, a lower user's vitality score may indicate a state that the user should avoid higher intensity of activity. For example, the electronic device 310 may identify a sleep heart rate and/or sleep heart rate variability and determine (or calculate) the user's vitality score based on the sleep heart rate and/or sleep heart rate variability. According to an embodiment, the electronic device 310 may produce (or output, determine, calculate) a higher vitality score as the sleep heart rate is lower and/or the sleep heart rate variability is higher. The electronic device 310 may produce (or output, determine, calculate) a lower vitality score as the sleep heart rate is higher and/or the sleep heart rate variability is lower. The electronic device 310 may determine a vitality score in consideration of the user's various information (e.g., the user's exercise history, the user's sleep time) as well as the sleep heart rate and/or sleep heart rate variability. For example, in case the user's sleep time is similar to the user's average sleep time, the electronic device 310 may produce (or output, determine, calculate) a higher vitality score. In case the user's exercise time is higher than the user's average exercise time, the electronic device 310 may produce (or output, determine, calculate) a lower vitality score. The electronic device 310 may display the determined vitality score on the display 160 or provide the determined vitality score to the user in various ways.

With reference to FIG. 8C, the electronic device 310 may display a screen 830 including information related to a vitality score on the display 160.

The screen 830 including information related to a vitality score may include sleep heart rate variability and sleep heart rate determined while the user was in the most recent sleep state, a vitality score 831 determined based on the user's sleep heart rate variability and sleep heart rate determined during a designated time period, information 832 indicating a difference value between average of the user's vitality scores determined during a designated time period (e.g., 1 week) and the vitality score 831, a description 833 of the vitality score 831, and/or an indicator 834 indicating a position of the determined vitality score 831 within the range of overall vitality scores.

The electronic device 310 may provide a screen 830 including information related to a vitality score to the user and induce the user to perform operations of identifying the vitality score and/or regulating the user's physical activity with reference to the screen 830 including information related to the vitality score.

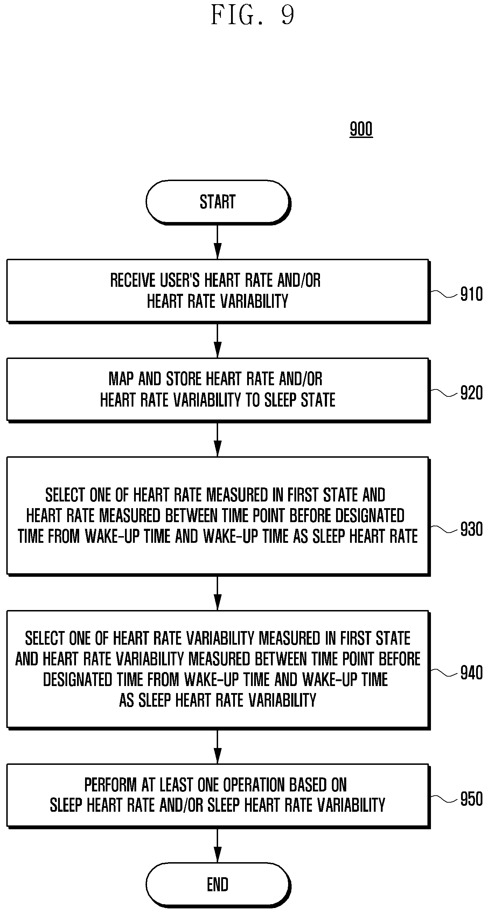

FIG. 9 is a flowchart 900 illustrating a method of operating an electronic device according to an embodiment.

At operation 910, the electronic device (e.g., the electronic device 310 of FIG. 3) may receive the user's heart rate and/or heart rate variability.

The electronic device 310 may receive the user's heart rate and/or heart rate variability measured by the external electronic device 320 from the external electronic device 320.

The external electronic device 320 may measure a heart rate and/or heart rate variability at each designated time (or cycle) in a state in which the external electronic device 320 is worn on a part (e.g., wrist and/or finger) of the user's body.

The heart rate variability may be an indicator representing changes in a time interval between heart beats. The heart rate variability may be a value related to the user's physical health. According to an embodiment, the user's body may be in a healthier state if the heart rate variability is high. Conversely, the user's body may be in a relatively unhealthy state if the heart rate variability is low.

A first sensor (e.g., biometric sensor) included in the external electronic device 320 emits (or outputs) an optical signal to the user's body and receives and/or analyzes a signal reflected by the user's body; thus, the external electronic device 320 may measure (or acquire) the user's heart rate and/or heart rate variability.

At operation 920, the electronic device 310 may map and store a heart rate and/or heart rate variability to the user's sleep state.

The electronic device 310 may receive the heart rate and/or heart rate variability transmitted by the external electronic device 320 and store the heart rate and/or the heart rate variability in the memory 430.

According to an embodiment, the electronic device 310 may store mapping data that maps the user's state determined based on the user's movement and/or heart rate and the heart rate and/or heart rate variability measured in the determined state in the memory 430.

The user's state may include the user's sleep state. Specifically, the electronic device 310 may map a heart rate and/or heart rate variability measured by the external electronic device 320 while the user is sleeping to the user's sleep state and store mapping data in the memory 430.

According to an embodiment, the user's sleep state may be distinguished according to the degree of movement of the sleeping user. For example, the user's sleep state may include a deep sleep state, which is a state in which the user's movement is lowest and in which the user's heart rate is relatively low, a REM sleep state, which is a state in which the user's movement is lowest and in which the user's heart rate is relatively high, a shallow sleep state, which is a state in which the degree of movement of the sleeping user is relatively high, and/or a state of being awakened during sleep, which is a state in which the degree of movement of the sleeping user is relatively high and in which the user's heart rate is relatively high. Four sleep states described above are only examples and may be implemented in various ways by the user and/or the manufacturer of the electronic device 310.

The electronic device 310 may receive information indicating a level of movement of the user measured by the external electronic device 320 through short-range wireless communication and distinguish (or determine, configure) sleep stages based on the user's level of movement.

According to an embodiment, in case the electronic device 310 is worn on at least a part of the user's body, the electronic device 310 may distinguish (or determine, configure) sleep stages based on information indicating a level of movement of the user measured thereby.

The electronic device 310 may distinguish a sleep state during the user's sleep time and store mapping data in which a heart rate and/or heart rate variability measured in each distinguished sleep state are/is mapped to the sleep state in the memory 430.

At operation 930, the electronic device 310 may select one of a heart rate measured in a first state (e.g., deep sleep state) and a heart rate measured between a time point before a designated time from a wake-up time and the wake-up time as a sleep heart rate.

The electronic device 310 may determine any one of at least one heart rate measured while the use is sleeping as a sleep heart rate (sleep HR) indicating a representative heart rate (or the most reliable heart rate) of heart rates measured during sleep with reference to mapping data (or heart rate and/or heart rate variability).

Determining (or selecting) a sleep heart rate may be for generating and/or providing accurate (or reliable) information related to the user's body based on the sleep heart rate representing the measured heart rates.

The electronic device 310 may determine any one of a heart rate measured in a first state (e.g., deep sleep state) among the measured heart rates and a heart rate (average of heart rates) measured from a time point before a designated time (e.g., 5 to 30 minutes) from the user's wake up time to the wake up time as a sleep heart rate.

In the case of a user with a specific pattern among users, a heart rate measured from a time point before a designated time from the wake-up time to the wake-up time than a heart rate measured in a deep sleep state may be a more reliable heart rate in representing the user's physical health. The specific pattern may include a pattern with a lot of activity amount before the user enters sleep. In the case of a user who has a lot of activity amount before a designated sleep time (e.g., 1-3 hours), a heart rate measured in a deep sleep state may be higher than a heart rate measured from a time point before a designated time from the wake-up time to the wake-up time, and a heart rate measured from a time point before a designated time from the wake-up time to the wake-up time may be a more reliable heart rate in representing the user's physical health.

The electronic device 310 may compare a heart rate measured in a deep sleep state among the measured heart rates and a heart rate (average of heart rates) measured from a time point before a designated time (e.g., 5 to 30 minutes) from the user's wake up time to the wake up time and determine (or select) a heart rate with a lower magnitude as the sleep heart rate.

According to an embodiment, the electronic device 310 may determine (or select) a heart rate (or average of heart rates) measured from a time point before a designated time (e.g., 5 to 30 minutes) from the user's wake up time to the wake up time as a sleep heart rate. The electronic device 310 may monitor (or identify) the user's activity amount before a designated time for the user to go to bed, and in case the user's activity amount satisfies a designated condition (e.g., a condition in which the user's activity amount is greater than or equal to a designated magnitude), the electronic device 310 may determine (or select) a heart rate (or average of heart rates) measured from a time point before a designated time (e.g., 5 to 30 minutes) from the user's wake up time to the wake up time as a sleep heart rate. In case the user's activity amount satisfies a designated condition (e.g., a condition in which the user's activity amount is greater than or equal to a designated magnitude), the electronic device 310 may not identify the user's heart rate during sleep.

At operation 940, the electronic device 310 may select one of heart rate variability measured in a first state and heart rate variability measured between a time point before a designated time from the wake-up time and the wake-up time as sleep heart rate variability.

The electronic device 310 may determine any one of heart rate variability measured in a first state (e.g., deep sleep state) among the measured heart rate variability and heart rate variability (or average of heart rate variability) measured from a time point before a designated time (e.g., 5 to 30 minutes) from the user's wake up time to the wake up time as sleep heart rate variability.

In the case of a user with a specific pattern among users, heart rate variability measured from a time point before a designated time from the wake-up time to the wake-up time than heart rate variability measured in a deep sleep state may be more reliable heart rate variability in representing the user's physical health. The specific pattern may include a pattern with a lot of activity amount before the user enters sleep. In the case of a user who has a lot of activity amount before a designated sleep time (e.g., 1-3 hours), heart rate variability measured in a deep sleep state may be lower than heart rate variability measured from a time point before the designated time from the wake-up time to the wake-up time, and heart rate variability measured from a time point before a designated time from the wake-up time to the wake-up time may be more reliable heart rate variability in representing the user's physical health.

The electronic device 310 may compare heart rate variability measured in a deep sleep state among the measured heart rate variability and heart rate variability (or average of heart rate variability) measured from a time point before a designated time (e.g., 5 to 30 minutes) from the user's wake up time to the wake up time and determine (or select) heart rate variability with a larger magnitude as sleep heart rate variability.

According to an embodiment, the electronic device 310 may determine (or select) heart rate variability (or average of heart rate variability) measured from a time point before a designated time (e.g., 5 to 30 minutes) from the user's wake up time to the wake up time as sleep heart rate variability. The electronic device 310 may monitor (or identify) the user's activity amount before a designated time for the user to go to bed, and in case the user's activity amount satisfies a designated condition (e.g., a condition in which the user's activity amount is greater than or equal to a designated magnitude), the electronic device 310 may determine (or select) heart rate variability (or average of heart rate variability) measured from a time point before a designated time (e.g., 5 to 30 minutes) from the user's wake up time to the wake up time as sleep heart rate variability. In case the user's activity amount satisfies a designated condition (e.g., a condition in which the user's activity amount is greater than or equal to a designated magnitude), the processor 420 may not identify the user's heart rate variability during sleep.

At operation 950, the electronic device 310 may perform at least one operation based on the sleep heart rate and/or sleep heart rate variability.

The electronic device 310 may perform at least one operation based on the sleep heart rate and/or sleep heart rate variability.

At least one operation based on the sleep heart rate and/or the sleep heart rate variability may include an operation of displaying (or outputting) the sleep heart rate and/or the sleep heart rate variability.

According to an embodiment, the electronic device 310 may display the sleep heart rate and/or the sleep heart rate variability on a display (e.g., the display module 160 of FIG. 1).

The sleep heart rate displayed on the display 160 may be displayed as in a heart rate according to the user's sleep time and be displayed along with the history of the sleep heart rate.

The sleep heart rate variability displayed on the display 160 may be displayed as in sleep heart rate variability according to the user's sleep time and be displayed along with the history of the sleep heart rate variability.

At least one operation based on the sleep heart rate and/or the sleep heart rate variability may include an operation of providing information related to the user's vitality state determined (or generated) based on the sleep heart rate and/or the sleep heart rate variability.

Information related to the user's vitality state may include a score that quantifies the user's vitality state. The score that quantifies a user's vitality state may be referred to as a vitality score. A higher user's vitality score may indicate a state that the user may perform higher intensity of activity. Conversely, a lower user's vitality score may indicate a state that the user should avoid higher intensity of activity. For example, the electronic device 310 may identify a sleep heart rate and/or sleep heart rate variability and determine (or calculate) the user's vitality score based on the sleep heart rate and/or the sleep heart rate variability. According to an embodiment, the electronic device 310 may produce (or output, determine, calculate) a higher vitality score as the sleep heart rate is lower and/or the sleep heart rate variability is higher. The electronic device 310 may determine a vitality score in consideration of the user's various information (e.g., user's exercise history, user's sleep time) as well as the sleep heart rate and/or the sleep heart rate variability. For example, in case the user' sleep time is similar to the user' average sleep time, the electronic device 310 may produce (or output, determine, calculate) a higher vitality score. In case the user's exercise time is higher than the user's average exercise time, the electronic device 310 may produce (or output, determine, calculate) a lower vitality score. The electronic device 310 may display the determined vitality score on the display 160 or may provide the determined vitality score to the user in various ways.

Information related to the user's vitality state may include information that may be used as a reference for the user to perform activity after waking up. For example, information related to the user's vitality state may include information that guides the user to perform appropriate intensity of activity after waking up. For example, the electronic device 310 may identify a sleep heart rate and/or sleep heart rate variability and determine whether the user's vitality state is sufficient to perform high intensity of activity. The electronic device 310 may identify that the user's vitality state is sufficient to perform high intensity of activity and provide a suggestion to the user to perform high intensity of activity in various forms (e.g., screen displayed on the display 160, sound output through the speaker). The electronic device 310 may identify that the user's vitality state is difficult to perform high intensity of activity and provide a suggestion to the user to perform low intensity of activity in various forms (e.g., screen displayed on the display 160, sound output through the speaker).

Information related to the user's vitality state may include guide information that enables the user to manage a heart rate and/or heart rate variability. The electronic device 310 may display guide information related to a sleep time on the display 160. Alternatively, in the case of a high heart rate and/or low heart rate variability, the electronic device 310 may display guide information that may guide activities that may reduce a user stress (e.g., yoga, meditation, deep breathing activities, activities that enable progressive muscle relaxation) on the display 160. Alternatively, in the case of a high heart rate and/or low heart rate variability, the electronic device 310 may display guide information that may guide activities that may lower a heart rate (e.g., regular low intensity exercise) on the display 160. In the case of a high heart rate and/or low heart rate variability, the electronic device 310 may display guidance information on lifestyle habits that may lower a heart rate (e.g., diet information that may lower a heart rate, diet information that increases a heart rate) on the display 160.

An electronic device 310 according to an embodiment may include a communication circuit 410 configured to communicate with an external electronic device 320 configured to measure movements and/or bio-signals of a user thereof. The electronic device 310 may include a processor 420. The electronic device 310 may include a memory 430. When executed by the processor 420, the memory 430 may store an instruction for causing the electronic device 310 to receive the user's heart rate (HR) and/or heart rate variability (HRV) measured by the external electronic device 320. The memory 430 may store an instruction for causing the electronic device 310 to map and store the heart rate and/or the heart rate variability to the user's sleep state determined based on the user's movement measured by the external electronic device 320. The memory 430 may store an instruction for causing the electronic device 310 to select one of the heart rate measured in a first state among the sleep states and a heart rate measured between a time point before a designated time from the user's wake up time and the wake up time as a sleep heart rate. The memory 430 may store an instruction for causing the electronic device 310 to select one of the heart rate variability measured in a first state among the sleep states and heart rate variability measured between a time point before a designated time from the user's wake up time and the wake up time as sleep heart rate variability. The memory 430 may store an instruction for causing the electronic device 310 to perform at least one operation based on the sleep heart rate and/or the sleep heart rate variability.

In the electronic device 310 according to an embodiment, the instruction for causing the electronic device 310 to select the sleep heart rate may include an instruction for causing the electronic device 310 to select a smaller heart rate of the heart rate measured in a first state among the sleep states and a heart rate measured between a time point before a designated time from the user's wake up time and the wake up time as a sleep heart rate.

In the electronic device 310 according to an embodiment, the instruction for causing the electronic device 310 to select the heart rate variability may include an instruction for causing the electronic device 310 to select larger heart rate variability of the heart rate variability measured in a first state among the sleep states and heart rate variability measured between a time point before a designated time from the user's wake up time and the wake up time as sleep heart rate variability.

In the electronic device 310 according to an embodiment, the first state may include a state in which the user's movement measured by the external electronic device 320 is smallest.

In the electronic device 310 according to an embodiment, the memory 430 may further store an instruction for causing the electronic device 310 to distinguish sleep stages according to the user's movement measured by the external electronic device 320 and/or the user's heart rate measured by the external electronic device 320. The memory 430 may further store an instruction for causing the electronic device 310 to store data in which the heart rate and/or the heart rate variability are/is mapped to the distinguished sleep stages therein.

In the electronic device 310 according to an embodiment, the at least one operation may include an operation of displaying the sleep heart rate and/or the sleep heart rate variability.

In the electronic device 310 according to an embodiment, the at least one operation may include an operation of providing information related to the user's vitality state determined based on the sleep heart rate and/or the sleep heart rate variability.

In the electronic device 310 according to an embodiment, the information related to the user's vitality state may include guide information based on the user's vitality state.

In the electronic device 310 according to an embodiment, the information related to the user's vitality state may include a vitality score indicating the user's vitality state.

A method of operating an electronic device according to an embodiment may include receiving the user's heart rate (HR) and/or heart rate variability measured by an external electronic device 320 configured to measure the user's movements and/or bio-signals of an electronic device 310. The method of operating an electronic device may include mapping the heart rate and/or the heart rate variability to the user's sleep state determined based on the user's movement measured by the external electronic device 320 and storing the heart rate and/or the heart rate variability in a memory 430. The method of operating the electronic device may include selecting one of the heart rate measured in a first state among the sleep states and a heart rate measured between a time point before a time designated from the user's wake up time and the wake up time as a sleep heart rate. The method of operating the electronic device may include selecting one of the heart rate variability measured in a first state among the sleep states and heart rate variability measured between a time point before a designated time from the user's wake up time and the wake up time as sleep heart rate variability. The method of operating the electronic device may include performing at least one operation based on the sleep heart rate and/or the sleep heart rate variability.

In a method of operating an electronic device according to an embodiment, selecting the sleep heart rate may include selecting a smaller heart rate of the heart rate measured in a first state among the sleep states and a heart rate measured between a time point before a designated time from the user's wake up time and the wake up time as a sleep heart rate.

In a method of operating an electronic device according to an embodiment, selecting the heart rate variability may include selecting larger heart rate variability of the heart rate variability measured in a first state among the sleep states and heart rate variability measured between a time point before a designated time from the user's wake up time and the wake up time as sleep heart rate variability.

In a method of operating an electronic device according to an embodiment, the first state may include a state in which the user's movement measured by the external electronic device 320 is smallest.

The method of operating an electronic device according to an embodiment may further include distinguishing sleep stages according to the user's movement measured by the external electronic device 320 and/or the user's heart rate measured by the external electronic device 320. The method of operation an electronic device may further include storing data in which the heart rate and/or the heart rate variability is/are mapped to the distinguished sleep stages in the memory 430.

In a method of operating an electronic device according to an embodiment, the at least one operation may include displaying the sleep heart rate and/or the sleep heart rate variability.

In a method of operating an electronic device according to an embodiment, the at least one operation may include providing information related to the user's vitality state determined based on the sleep heart rate and/or sleep heart rate variability.

In a method of operating an electronic device according to an embodiment, the information related to the user's vitality state may include guide information based on the user's vitality state.

In a method of operating an electronic device according to an embodiment, the information related to the user's vitality state may include a vitality score indicating the user's vitality state.

When executed by a processor 420 of an electronic device according to an embodiment, in a computer readable recording medium for storing instructions for causing the electronic device 310 to perform an electronic device, the instructions may include an instruction for causing the electronic device 310 to receive a user's heart rate (HR) and/or heart rate variability measured by an external electronic device 320. The instructions may include an instruction for causing the electronic device 310 to map and store the heart rate and/or heart rate variability to the user's sleep state determined based on the user's movement measured by the external electronic device 320. The instructions may include an instruction for causing the electronic device 310 to select one of the heart rate measured in a first state among the sleep states and a heart rate measured between a time point before a designated time from the user's wake up time and the wake up time as a sleep heart rate. The instructions may include an instruction for causing the electronic device 310 to select one of the heart rate variability measured in a first state among the sleep states and heart rate variability measured between a time point before a designated time from the user's wake up time and the wake up time as sleep heart rate variability. The instructions may include an instruction for causing the electronic device 310 to perform at least one operation based on the sleep heart rate and/or the sleep heart rate variability.

In a recording medium according to an embodiment, the instruction for causing the electronic device 310 to select the sleep heart rate may include an instruction for causing the electronic device 310 to select a smaller heart rate of a heart rate measured in a first state among the sleep states and a heart rate measured between a time point before a designated time from the user's wake up time and the wake up time as a sleep heart rate.

According to an embodiment, an electronic device includes a communication circuit configured to communicate with an external electronic device, a processor and a memory having executable instructions stored thereon. When the executable instructions are executed by the processor, the electronic device is caused to execute a method. The method includes receiving a heart rate (HR) and heart rate variability (HRV) of a user measured by the external electronic device, determining sleep states of the user based on user movement measured by the external electronic device, mapping the HR and the HRV to the sleep states, selecting, as a sleep HR, one of the HR measured in a first state among the sleep states and the HR measured between a designated time and a wake-up time of the user, selecting, as a sleep HRV, one of the HRV measured in the first state among the sleep states and the HRV measured between the designated time and the wake-up time and performing an operation based on the sleep HR and the sleep HRV.

According to an embodiment of the electronic device, the selecting, as the sleep HR, the one of the HR measured in the first state among the sleep states and the HR measured between the designated time and the wake-up time of the user comprises selecting the smaller HR.

According to an embodiment of the electronic device, the selecting, as the sleep HRV, the one of the HRV measured in the first state among the sleep states and the HRV measured between the designated time and the wake-up time instruction comprises selecting the larger HRV.

According to an embodiment of the electronic device, the first state comprises a state in which the user movement measured by the external electronic device is smallest.

According to an embodiment of the electronic device, the method further comprises: distinguishing sleep stages according to one of the user movement measured by the external electronic device and the HR of the user measured by the external electronic device, and storing data in which the HR and the HRV are mapped to the sleep stages in the memory.

According to an embodiment of the electronic device, the operation comprises displaying the sleep HR and the sleep HRV.

According to an embodiment of the electronic device, the operation comprises providing information related to a vitality state of the user determined based on the sleep HR and the sleep HRV.

According to an embodiment of the electronic device, the information related to the vitality state of the user comprises guide information.

According to an embodiment of the electronic device, the information related to the vitality state of the user comprises a vitality score.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device, comprising:
a communication circuit configured to communicate with an external electronic device configured to measure movements and/or bio-signals of a user of the electronic device;
a processor; and
a memory,
wherein when executed by the processor, the memory is configured to store instructions for causing the electronic device to:
receive the user's heart rate and/or heart rate variability measured by the external electronic device,
map and store the heart rate and/or the heart rate variability to the user's sleep state determined based on the user's movement measured by the external electronic device,
select one of the heart rate measured in a first state among the sleep states and the heart rate measured between a time point before a designated time from the user's wake up time and the wake up time as a sleep heart rate,
select one of the heart rate variability measured in the first state among the sleep states and the heart rate variability measured between the time point before the designated time from the user's wake up time and the wake up time as sleep heart rate variability, and
perform at least one operation based on the sleep heart rate and/or the sleep heart rate variability.

2. The electronic device of claim 1, wherein the instruction for causing the electronic device to select the heart rate comprises an instruction for causing the electronic device to select a smaller heart rate of the heart rate measured in a first state among the sleep states and a heart rate measured between a time point before a designated time from the user's wake up time and the wake up time as a sleep heart rate.

3. The electronic device of claim 1, wherein the instruction for causing the electronic device to select the heart rate variability comprises an instruction for causing the electronic device to select larger heart rate variability of the heart rate variability measured in a first state among the sleep states and heart rate variability measured between a time point before a designated time from the user's wake up time and the wake up time as sleep heart rate variability.

4. The electronic device of claim 1, wherein the first state comprises a state in which the user's movement measured by the external electronic device is smallest.

5. The electronic device of claim 1, wherein the memory is configured to further store an instruction for causing the electronic device to:
distinguish sleep stages according to the user's movement measured by the external electronic device and/or the user's heart rate measured by the external electronic device, and
store data in which the heart rate and/or the heart rate variability are/is mapped to the distinguished sleep stages in the memory.

6. The electronic device of claim 1, wherein the at least one operation comprises an operation of displaying the sleep heart rate and/or the sleep heart rate variability.

7. The electronic device of claim 1, wherein the at least one operation comprises an operation of providing information related to the user's vitality state determined based on the sleep heart rate and/or the sleep heart rate variability.

8. The electronic device of claim 7, wherein the information related to the user's vitality state comprises guide information based on the user's vitality state.

9. The electronic device of claim 7, wherein the information related to the user's vitality state comprises a vitality score indicating the user's vitality state.

10. A method of operating an electronic device, the method comprising:
receiving a user's heart rate and/or heart rate variability measured by an external electronic device configured to measure the user's movements and/or bio-signals of the electronic device;
mapping the heart rate and/or the heart rate variability to the user's sleep state determined based on the user's movement measured by the external electronic device and storing the heart rate and/or the heart rate variability in a memory;
selecting one of the heart rate measured in a first state among the sleep states and the heart rate measured between a time point before a time designated from the user's wake up time and the wake up time as a sleep heart rate;
selecting one of the heart rate variability measured in the first state among the sleep states and the heart rate variability measured between the time point before the designated time from the user's wake up time and the wake up time as sleep heart rate variability; and
performing at least one operation based on the sleep heart rate and/or the sleep heart rate variability.

11. The method of claim 10, wherein selecting the sleep heart rate comprises selecting a smaller heart rate of a heart rate measured in a first state among the sleep states and a heart rate measured between a time point before a designated time from the user's wake up time and the wake up time as a sleep heart rate.

12. The method of claim 10, wherein selecting the heart rate variability comprises selecting larger heart rate variability of the heart rate variability measured in a first state among the sleep states and heart rate variability measured between a time point before a designated time from the user's wake up time and the wake up time as sleep heart rate variability.

13. The method of claim 10, wherein the first state comprises a state in which the user's movement measured by the external electronic device is smallest.

14. The method of claim 10, further comprising:
distinguishing sleep stages according to the user's movement measured by the external electronic device and/or the user's heart rate measured by the external electronic device; and
storing data in which the heart rate and/or the heart rate variability is/are mapped to the distinguished sleep stages in the memory.

15. The method of claim 10, wherein the at least one operation comprises displaying the sleep heart rate and/or the sleep heart rate variability.
